(19) Europäisches Patentamt
European Patent Office
Office européen des brevets

(11) EP 3 717 447 B1

(12) **EUROPEAN PATENT SPECIFICATION**

(45) Date of publication and mention of the grant of the patent:
**15.05.2024 Bulletin 2024/20**

(21) Application number: **18825568.1**

(22) Date of filing: **30.11.2018**

(51) International Patent Classification (IPC):
*C07C 45/48* (2006.01) *C07C 29/145* (2006.01)
*C07C 41/03* (2006.01) *A01N 25/30* (2006.01)
*C08G 65/26* (2006.01) *C07C 31/125* (2006.01)
*C07C 43/11* (2006.01) *C07C 49/76* (2006.01)
*C07C 49/04* (2006.01)

(52) Cooperative Patent Classification (CPC):
(C-Sets available)
**C07C 45/48; A01N 25/30; C07C 29/145; C07C 41/03; C08G 65/2609; C08G 65/2612; C09K 23/00; C09K 23/42** (Cont.)

(86) International application number:
**PCT/EP2018/083241**

(87) International publication number:
**WO 2019/106196 (06.06.2019 Gazette 2019/23)**

(54) **PROCESS FOR THE PREPARATION OF ALKOXYLATES COMPOSITIONS**

VERFAHREN ZUR HERSTELLUNG VON ALKOXYLATZUSAMMENSETZUNGEN

PROCÉDÉ DE PRÉPARATION DE COMPOSITIONS D'ALKOXYLATES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **01.12.2017 EP 17306685**

(43) Date of publication of application:
**07.10.2020 Bulletin 2020/41**

(73) Proprietor: **SPECIALTY OPERATIONS FRANCE**
**69003 Lyon (FR)**

(72) Inventors:
- **BACK, Olivier**
  **69008 Lyon (FR)**
- **BRAMATI, Valerio**
  **20020 Arese (IT)**

(74) Representative: **Vande Gucht, Anne et al**
**Solvay S.A.**
**Département de la Propriété Industrielle**
**Rue de Ransbeek, 310**
**1120 Bruxelles (BE)**

(56) References cited:
**EP-A1- 3 415 493**
**EP-A2- 0 850 907**
**WO-A1-2018/229285**
**CN-A- 106 220 478**
**US-A- 3 359 331**
**US-A- 3 372 201**
**US-A1- 2011 319 669**
**US-B1- 9 193 650**

- **LUKAS J. GOOSSEN ET AL: "Catalytic Decarboxylative Cross-Ketonisation of Aryl- and Alkylcarboxylic Acids using Magnetite Nanoparticles", ADVANCED SYNTHESIS & CATALYSIS, vol. 353, no. 1, 10 January 2011 (2011-01-10), pages 57-63, XP055076256, ISSN: 1615-4150, DOI: 10.1002/adsc.201000429**
- **IGNATCHENKO ALEXEY V ET AL: "Cross-selectivity in the catalytic ketonization of carboxylic acids", APPLIED CATALYSIS A: GENERAL, ELSEVIER, AMSTERDAM, NL, vol. 498, 24 March 2015 (2015-03-24), pages 10-24, XP029220083, ISSN: 0926-860X, DOI: 10.1016/J.APCATA.2015.03.017**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 717 447 B1

(52) Cooperative Patent Classification (CPC): (Cont.)

C-Sets
**A01N 25/30, A01N 25/02;**
**A01N 25/30, A01N 25/04;**
**A01N 25/30, A01N 53/00;**
**A01N 25/30, A01N 57/16;**
**C07C 29/145, C07C 31/125;**
**C07C 29/145, C07C 33/20;**
**C07C 41/03, C07C 43/11;**
**C07C 41/03, C07C 43/1785;**
**C07C 45/48, C07C 49/04;**
**C07C 45/48, C07C 49/76**

## Description

### CROSS-REFERENCE TO A RELATED APPLICATION

This application claims priority to European application No. 17306685.3 filed on December 1, 2017.

### TECHNICAL FIELD

The technical field of the present invention relates to non-ionic surfactants and in particular, alkoxylates surfactants. Alkoxylates surfactants have the property to stabilize emulsions and dispersions used in agricultural or pharmaceutical formulations.

### BACKGROUND ART

Among non-ionic surfactants that are widely used, one may mention nonylphenol ethoxylates (NPE) and tristyrylphenol ethoxylates (TSE), the structure of which is shown hereafter.

NPE:

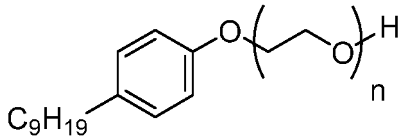

TSE:

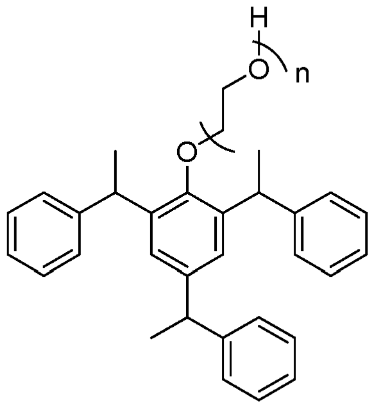

Both compounds contain at least one phenyl ring and a polyalkoxylated chain directly attached thereto.
NPE-type compounds are cheap and versatile surfactants. They display good wetting, emulsifying and dispersing properties.
As an example of composition containing a NPE-type compound, US 4,233,174 discloses a cleaning composition comprising from 35 to 80 wt. % of one or both of:

- a fatty alcohol ethoxylate having from 8 to 18 carbon atoms in the alkyl chain and resulting from the condensation of from 2 to 10 moles of ethylene oxide per mole of alcohol and
- an alkyl phenol ethoxylate, that is a NPE-type compound, having from 8 to 12 carbon atoms in the alkyl moiety and resulting from the condensation of from 4 to 10 moles of ethylene oxide per mole of phenol.

However, one drawback of NPE-type compounds is that they are not readily biodegradable. Further, their precursor and degradation product nonylphenol is classified as an endocrine disruptor and is reprotoxic. For this reason, NPE-type compounds have been banned in Europe, but they are still used in the US and in Asia.
In addition, TSE-type compounds are not readily biodegradable either. Due to their bio-accumulating behavior they are considered as a persistent organic pollutant. However, they are widely used in agricultural formulations (for example in emulsifiable concentrates and suspension concentrates) due to their excellent emulsifying and dispersing

properties.

There is therefore a need to find alternatives to NPE and TSE that are less toxic, possessing a better environmental profile, that is to say, that have enhanced biodegradability and exhibit no endocrine disruptor and no reprotoxicity properties.

Alkyl ethoxylates that are less toxic are often used as a substitute to NPE. However, they are not as efficient as emulsifiers as NPE and they are also more expensive.

Document CN 106220478 describes an alpha-phenyl alkyl alcohol poly(oxyethylene) ether as a substitute to NPE. The alpha-phenyl alkyl alcohol poly(oxyethylene) ether has the formula:

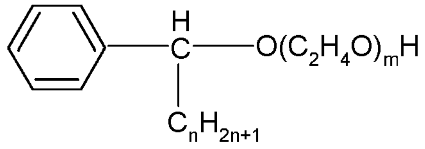

where n ranges from 5 to 18 and m ranges from 3 to 50.

It is mentioned that this non-ionic surfactant displays good wetting, infiltrating, emulsifying, dispersing, solubilizing and washing properties and can be used as an emulsifier, a textile auxiliary, a detergent, a dispersant, a softener, a crude oil demulsifier and can replace alkylphenol ethoxylate.

Documents CN106279670, CN106187834, CN106220679 describe derivatives of the alpha-phenyl alkyl alcohol poly(oxyethylene) ether disclosed in CN 106220478. These derivatives are the ether sulfate derivative, the disodium succinate derivative and the ether phosphate.

In CN106220478 the process for synthesizing alpha-phenyl alkyl alcohol poly(oxyethylene) ether is based on the following steps:

1) a Friedel-Craft acylation between a fatty acyl chloride and benzene using stoichiometric quantities of Lewis acid ($AlCl_3$);
2) Hydrogenation under hydrogen pressure using a transition metal-based catalyst;
3) Ethoxylation of the carbinol obtained in step 2.

This process affords pure alpha-phenyl alkyl alcohol poly(oxyethylene) ether. However, it involves the use of carcinogenic, mutagenic or toxic for reproduction ("CMR") reactants, such as benzene. In addition, it requires the initial transformation of fatty acid into acyl chloride adding an extra-step which is also source of by-products and wastes generation. It usually requires the use of harmful reactants such as $PCl_3$, $SOCl_2$ etc.

The Friedel-Craft acylation requires the use of stoichiometric amounts of harmful Lewis acids such as $AlCl_3$ and therefore generates an important quantity of salts.

In conclusion, the process described in CN106220478 has very low sustainability: low atom economy (which is defined as the ratio between the molecular weight of the product divided by sum of the molecular weights of the reactants multiplied by the stoichiometry, high E-factor which is defined as the total mass of generated wastes per ton of desired product) and toxicity. It is not economically relevant. There is thus a need to find an alternative process for the preparation of the alpha-phenyl alkyl alcohol poly(oxyethylene) ether.

Secondary alcohol ethoxylates (SAEO) are known as surfactants. One may cite the commercial product Tergitol(™) 15-S-5 mainly comprising the secondary alcohol ethoxylate having the formula:

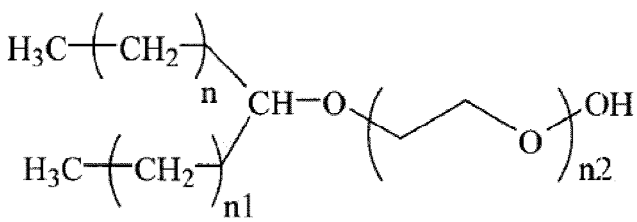

where n+n1 =15 and n2 = 4

It is known to prepare a mixture comprising a NPE-type compound and a secondary alcohol ethoxylate. For example, US 6,846,793 discloses a cleaning concentrate comprising a surfactant having an HLB value from 1 to 10 (HLB: hydrophilic-lipophilic balance), such as a secondary alcohol ethoxylate, and a NPE-type compound of formula:

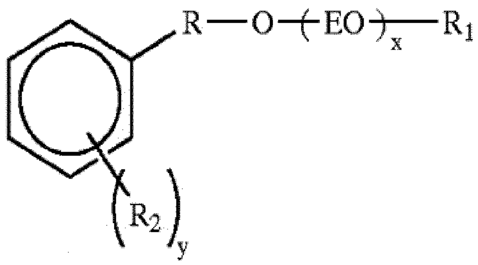

wherein:

x is an integer from 2 to 6;
y is an integer from 0 to 5;
R can be a bond or ($C_1$-$C_4$) alkylene;
$R_1$ is a hydrogen, halo, aryl, ($C_1$-$C_4$)alkyl, heteroaryl, cycloalkyl, or heterocycle;
$R_2$ is independently selected from hydrogen, halo, ($C_1$-$C_4$)alkyl, ($C_1$-$C_4$)alkoxy, ($C_2$-$C_4$) alkenylene.

There is still a need to provide new non-ionic surfactants formulations free of NPE-type compounds and TSE-type compounds.
There is also a need to provide a non-toxic and economically viable process for the preparation of the alpha-phenyl alkyl alcohol poly(oxyethylene) ether disclosed in CN 106220478, as a substitute to NPE-type compounds. A process alternative to the Friedel-Crafts acylation disclosed in CN 106220478 is therefore sought.

## SUMMARY OF THE INVENTION

The present invention relates to a process for the preparation of a mixture (I) comprising:

- an aryl aliphatic carbinol alkoxylate of formula (A):

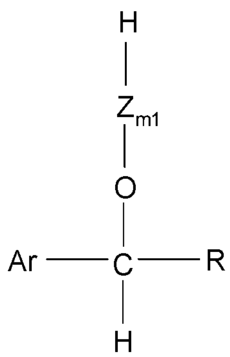

(A)

  where R is an aliphatic radical having from 4 to 24 carbon atoms; Z is an alkoxylate unit of the general formula -(Y-O)- where Y is a divalent hydrocarbon radical containing from 2 to 6 carbon atoms and 50≥m1≥2; Ar is an aryl radical;
- a dialiphatic carbinol alkoxylate of formula (B):

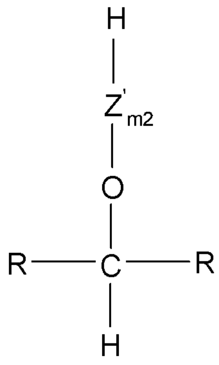

(B)

where

R is the same as above;
Z' is an alkoxylate unit of the general formula -(Y'-O)- where Y' is a divalent hydrocarbon radical containing from 2 to 6 carbon atoms and Z' can be the same as Z or can be different from Z and 50≥m2≥2, said process comprising the steps of:

a) performing a catalytic decarboxylative cross-ketonisation of aryl- and aliphatic carboxylic acids comprising the steps of:

i) providing a mixture containing:

- an aryl carboxylic acid of formula

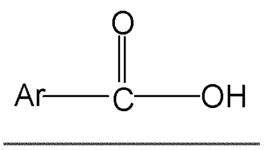

where Ar is the same as defined above;

- an aliphatic carboxylic acid of formula

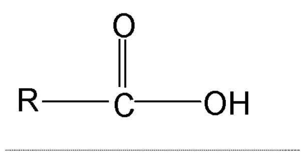

where R is the same as defined above;

- a catalyst;

ii) heating the mixture at a temperature sufficient to form mixture (III) comprising a dialiphatic ketone of formula (F)

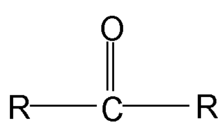

(F)

where R is the same as defined above

and an aryl aliphatic ketone of formula (G)

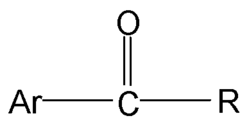 (G)

where R and Ar are the same as defined above;

b) subjecting mixture (III) to a hydrogenation step in the presence of a base to form mixture (II) comprising:

- an aryl aliphatic carbinol of formula (D):

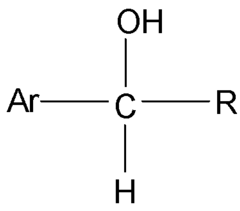 (D)

where

R is an aliphatic radical having from 4 to 24 carbon atoms;

Ar is an aryl radical;

- a dialiphatic carbinol of formula (E):

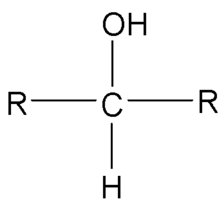 (E)

where R is the same as defined above;

c) subjecting mixture (II) to an alkoxylation step to form mixture (I);
wherein step a) of performing the catalytic decarboxvlative cross-ketonisation of aryl- and aliphatic carboxylic acids comprises the steps of:

i) providing a mixture containing:

- the aryl carboxylic acid;

- the aliphatic carboxylic acid;

- a metal-containing compound;

in which the number of moles of the metal in the mixture is at least equal to 90% of the sum of the number of moles of aryl carboxylic acid and the number of moles of aliphatic carboxylic acid divided by the valency of the metal

said mixture being free of any added solvent;

ii) heating the mixture at a temperature sufficient to form metal carboxylates;

iii) further heating the mixture at a temperature sufficient to form the dialiphatic ketone of formula (F) and the aryl aliphatic ketone of formula (G);

iv) adding to the reaction mixture of step iii):

- aryl carboxylic acid in an amount which corresponds substantially to the amount of aryl carboxylic acid consumed during the formation of the aryl aliphatic ketone in step iii);

- aliphatic carboxylic acid in an amount which corresponds substantially to the amount of aliphatic carboxylic acid consumed during the formation of the aryl aliphatic ketone and the dialiphatic ketone in step iii);

maintaining the mixture at a temperature sufficient to continue forming the dialiphatic ketone and the aryl aliphatic ketone:
v) optionally repeating step iv).

In one embodiment, the aliphatic radical is an alkyl radical.
In one embodiment, the polyalkoxylated chain $-Z_{m1}-$ has formula (C) where $0 \leq x \leq 20$ ; $0 \leq y \leq 20$ and $2 \leq x+y \leq 40$;

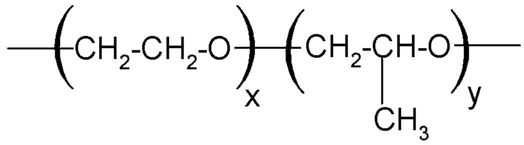

(C)

- the polyalkoxylated chain $-Z'_{m2}-$ has formula (C') where $0 \leq x' \leq 20$; $0 \leq y' \leq 20$ and $2 \leq x'+y' \leq 40$ ;

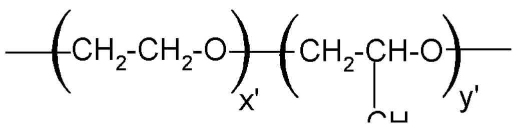

(C')

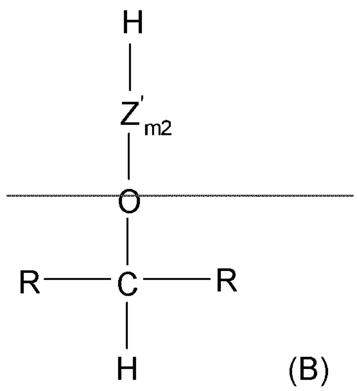

(B)

**DETAILED DESCRIPTION OF THE INVENTION**

In the following description, the term "aliphatic carboxylic acid" refers to a fatty acid, that is, a monocarboxylic acid having a long aliphatic chain. The aliphatic chain may be linear or branched, saturated or unsaturated. The aliphatic chain may be an alkyl chain. The total number of carbon atoms in the carboxylic fatty acid may vary from 4 to 28. Preferred fatty acids are those containing a number of carbon atoms from 8 to 22 carbon atoms, preferably from 12 to 18 carbon atoms. The most preferred fatty acids are caprylic acid ($C_8$), capric acid ($C_{10}$), lauric acid ($C_{12}$), myristic acid ($C_{14}$), palmitic acid ($C_{16}$), oleic and stearic acids ($C_{18}$).
The term "aryl radical" refers to a radical obtained from removing one hydrogen atom from an aromatic ring which can be optionally substituted by one or more hydrocarbon group and/or halogen. The hydrocarbon group can be optionally substituted and/or interrupted by a heteroatom or a heteroatom-containing group.
The term "aromatic ring" can designate a cyclic conjugated unsaturated hydrocarbon ring having a number of

delocalized π electrons following the Huckel rule.

The term "aromatic ring" encompasses also heteroaromatic ring, that is to say, cyclic conjugated unsaturated ring containing one or more heteroatoms and having a number of delocalized π electrons following the Huckel rule.

The term "aromatic ring" encompasses also polycyclic aromatic compounds. In the polycyclic aromatic compounds, the rings can be fused or they can be linked by C-C linkage or by a spiro linkage.

The term "aryl carboxylic acid" refers to a monocarboxylic acid having the formula

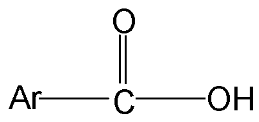

where Ar is an aryl radical as defined above. Aryl carboxylic acids may be selected from the group consisting of benzoic acid, toluic acid, furoic acid, nicotinic acid, thiophenecarboxylic acid, 2-mesitylenecarboxylic acid, isopropylbenzoic acid, chlorobenzoic acid, fluorobenzoic acid, trifluoromethylbenzoic acid, methoxybenzoic acid, naphthoic acid, anthracenecarboxylic acid, biphenylcarboxylic acid, benzoylbenzoic acid. The most preferred aryl carboxylic acids are benzoic acid and toluic acid.

The different chemicals and chemical steps involved in the present invention may be summarized as follows:

**1) Cross-ketonization (formation of ketone mixtures):**

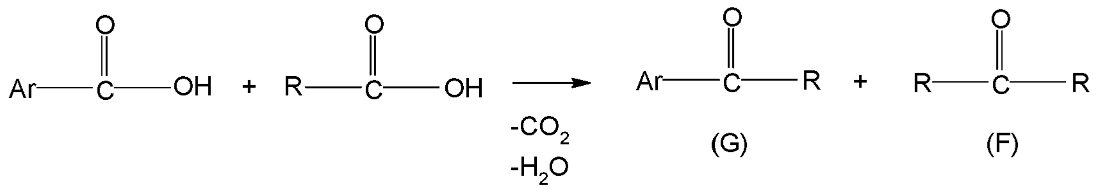


where:

- R is an aliphatic radical;
- Ar is an aryl radical

**2) Hydrogenation (formation of carbinol mixtures):**

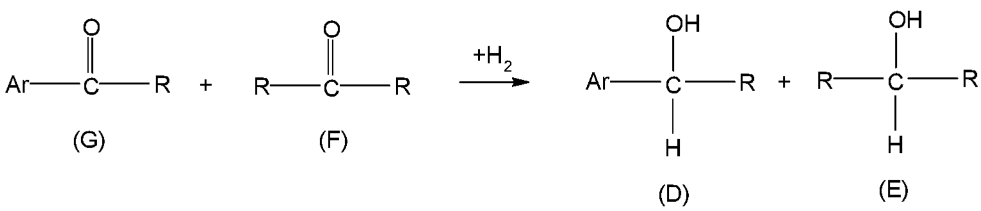


**3) Alkoxylation (formation of alkoxylate mixtures):**

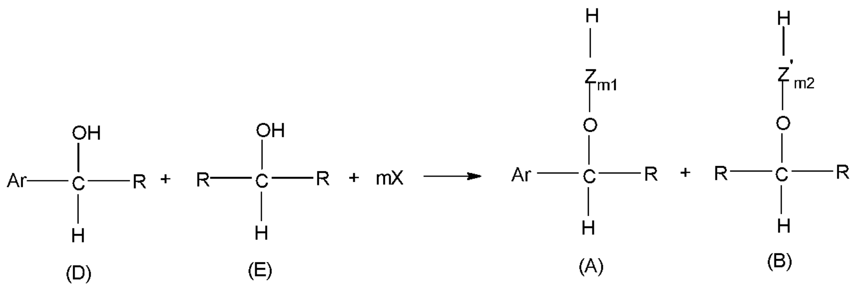

where X is an alkylene oxide containing from 2 to 6 carbon atoms and
Z is an alkoxylate unit of the general formula -(Y-O)- where Y is a divalent hydrocarbon radical containing from 2 to 6 carbon atoms that is derived from X;
Z' is an alkoxylate unit of the general formula -(Y'-O)- where Y' is a divalent hydrocarbon radical containing from 2 to 6 carbon atoms that is derived from X;
Z can be the same as Z' or different from Z';
$50 \geq m_1 \geq 2$ ; $50 \geq m_2 \geq 2$ and $m=m_1+m_2$.

The process for preparing the mixture of alkoxylates (A) and (B) preferably comprises the three following steps in this order: a catalytic decarboxylative cross-ketonisation, a hydrogenation and an alkoxylation.

The **first** step of the process is a catalytic decarboxylative cross-ketonisation of aryl carboxylic acids and aliphatic carboxylic acids, also called Piria reaction. Such a process is for example disclosed in EP-A-2 468 708. The catalytic decarboxylative cross-ketonisation of aryl carboxylic acids and aliphatic carboxylic acids typically comprises the following steps:

i) providing a mixture containing at least one aryl carboxylic acid Ar-C(=O)-OH, at least one aliphatic carboxylic acid of formula R-C(=O)-OH and a catalyst;
ii) heating the mixture at a temperature sufficient to form mixture (III) comprising a dialiphatic ketone of formula (F)

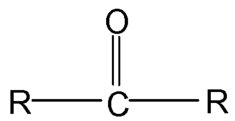
(F)

where R is the same as defined above

and an aryl aliphatic ketone of formula (G)

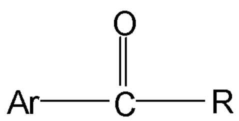
(G)

where R is the same as defined above.

$CO_2$ and water are continuously removed during the cross-ketonization.

In the above process, the mixture of ketones acts itself as a solvent. Therefore, no other solvent is needed to be added to the mixture of ketones.

According to the invention, the catalytic decarboxylative cross-ketonisation of aryl carboxylic acids and aliphatic carboxylic acids typically comprises the steps of:

a) providing a mixture containing:

i) the aryl carboxylic acid;
ii) the aliphatic carboxylic acid;
iii) a metal-containing compound;

in which the number of moles of the metal in the mixture is at least equal to 90% of the sum of the number of moles of aryl carboxylic acid and the number of moles of aliphatic carboxylic acid divided by the valency of the metal
said mixture being free of any added solvent;

b) heating the mixture at a temperature sufficient to form metal carboxylates;
c) further heating the mixture at a temperature sufficient to form the dialiphatic ketone and the aryl aliphatic ketone;
d) adding to the reaction mixture of step c):

i) aryl carboxylic acid in an amount which corresponds substantially to the amount of aryl carboxylic acid consumed during the formation of the aryl aliphatic ketone in step c);
ii) aliphatic carboxylic acid in an amount which corresponds substantially to the amount of aliphatic carboxylic acid consumed during the formation of the aryl aliphatic ketone and the dialiphatic ketone in step c);

maintaining the mixture at a temperature sufficient to continue forming the dialiphatic ketone and the aryl aliphatic ketone;
e) optionally repeating step d).

Suitable decarboxylative cross-ketonisation conditions may be as follows:

- assuming that x is the number of moles of aryl carboxylic acid and y is the number of moles of aliphatic carboxylic acid. The number of moles of a metal of valence z in the mixture of step a) may be within the range of from 0.9[(x+y)/z] to 1.1[(x+y)/z], preferably from 0.95 [(x+y)/z] to 1.05 [(x+y)/z], more preferably from 0.98[(x+y)/z] to 1.02[(x+y)/z], more preferably from 0.99[(x+y)/z] to 1.01[(x+y)/z].
- The metal-containing compound is preferably iron metal but it may also be selected from the group consisting of bivalent $Fe^{(II)}$ compounds, trivalent $Fe^{(III)}$ compounds, compounds in which iron is present in both bivalent $Fe^{(II)}$ and trivalent $Fe^{(III)}$ oxidation states, such as magnetite $Fe_3O_4$.
- step b) may be carried out at a pressure around 1 bar and at a temperature $T_1$ from 225°C to 290°C, preferably from 250°C to 275°C;
- step c) and/or step d) may be carried out at a pressure around 1 bar and at a temperature $T_2$ from 300°C to 400°C, preferably from 315°C to 400°C;
- the molar ratio of the total amount of aryl carboxylic acid and the aliphatic carboxylic acid may range from 0.3 to 1.8.
- heating in step b) may be carried out for a duration ranging from 1 to 6 h, preferably for a duration less than or equal to 3h;
- heating in step c) may be carried out for a duration ranging from 1 to 4 h, preferably for a duration less than or equal to 1.5h;
- heating in step d) may be carried out for a duration ranging from 1 to 4 h, preferably for a duration less than or equal to 1.25 h;
- The whole decarboxylative cross-ketonisation process may be carried out for a duration less than or equal to 20 hours, preferably for a duration less than or equal to 8 hours.

The aryl carboxylic acid may be selected from the group consisting of benzoic acid, furoic acid, o-toluic acid, m-toluic acid and p-toluic acid.

- The aliphatic carboxylic acid may be a fatty acid, preferably having from 8 to 18 carbon atoms.

The first step of the process leads to mixture (III), that is, a mixture comprising the dialiphatic ketone of formula (F) and the aryl aliphatic ketone of formula (G).
In mixture (III), the weight of the dialiphatic ketone of formula (F) based on the total weight of the dialiphatic ketone of formula (F) and of the aryl aliphatic ketone of formula (G), namely the ratio $r_{FG}$ with $r_{FG}$ being defined as follows:

$$r_{FG} = \text{weight of (F)} / [\text{ weight of (F)} + \text{weight of (G)} ]$$

can be of up to 2%, up to 5%, up to 10%, up to 20%, up to 30% or up to 50%.
Besides, the ratio $r_{FG}$ can be of at least 1%, at least 2%, at least 5%, at least 10%, or at least 20%.
Some preferred ranges for $r_{FG}$ are from 1% to 50%, from 1% to 20%, from 2% to 30% and from 2% to 20%.
In mixture (III), the total weight of the dialiphatic ketone of formula (F) and of the aryl aliphatic ketone of formula

(G) based on the total weight of the mixture (III) can be of at least 10%, at least 20%, at least 50%, at least at least 80% or at least 90%; it can also be of at least 95%, at least 98% or at least 99%. Further, mixture (III) can consist essentially of or can consist of the dialiphatic ketone of formula (F) and of the aryl aliphatic ketone of formula (G).

In the **second step** of the process, the mixture containing the dialiphatic ketone and the aryl aliphatic ketone undergoes a hydrogenation by which the dialiphatic ketone is converted into a dialiphatic alcohol, also referred hereinafter as dialiphatic carbinol, and by which the aryl aliphatic ketone is converted into an aryl aliphatic alcohol, also referred hereinafter as aryl aliphatic carbinol. The aryl aliphatic ketone is more reactive than the dialiphatic ketone under standard hydrogenation conditions. Thus, the conversion rate of the aryl aliphatic ketone is generally higher than that of the dialiphatic ketone. The dialiphatic ketone needs harsh hydrogenation conditions in order to obtain a suitable conversion rate. The hydrogenation temperature and the hydrogenation pressure are preferably selected in such a way that a conversion level of the dialiphatic ketone of at least 20% is obtained after 4 hours. A suitable hydrogenation temperature can be at least 120°C, preferably at least 150°C, even more preferably at least 180°C. A suitable hydrogenation pressure can be at least 10 bar, preferably at least 20 bar and preferably at least 30 bar. The duration of the hydrogenation may range from 0.5 to 10 hours, preferably from 2 to 6 hours, more preferably about 4 hours.

The hydrogenation catalyst is typically a platinum group metal supported on carbon. The loading of the platinum group metal generally ranges from 0.5 to 10 wt.%. Preferably, the loading ranges from 1 to 5 wt.%.

The hydrogenation is preferably conducted in the presence of a base, such as NaOH. The presence of such base significantly improves the selectivity toward the aryl aliphatic carbinol by reducing the formation of by-products that are formed during the hydrogenation through hydrodeoxygenation (e.g. alkylbenzene) and/or aromatic ring hydrogenation (e.g alkylcyclohexane) side reactions on the phenyl aliphatic ketone. The presence of such base also improves the conversion rate of the dialiphatic ketone as it will be shown in the experimental section. The base is added at a molar percentage generally ranging from 5 to 20% with respect to the sum of the number of moles of the ketones.

The second step of the process leads to mixture (II), that is a mixture comprising the dialiphatic carbinol and the aryl aliphatic carbinol. Mixture (II) may further contain residual amounts of the dialiphatic ketone and aryl aliphatic ketone which have not been hydrogenated.

In mixture (II), the weight of the dialiphatic carbinol of formula (E) based on the total weight of the aryl aliphatic carbinol of formula (D) and of the dialiphatic carbinol of formula (E), namely the ratio $r_{ED}$ with $r_{ED}$ being defined as follows:

$$r_{ED} = \text{weight of (E)} / [\text{ weight of (E)} + \text{weight of (D)} ]$$

can be of up to 2%, up to 5%, up to 10%, up to 20%, up to 30% or up to 50%.

Besides, the ratio $r_{ED}$ can be of at least 1%, at least 2%, at least 5%, at least 10%, or at least 20%.

Some preferred ranges for $r_{ED}$ are from 1% to 50%, from 1% to 20%, from 2% to 30% and from 2% to 20%.

In mixture (II), the total weight of the aryl aliphatic carbinol of formula (D) and of the dialiphatic carbinol of formula (E) based on the total weight of the mixture (II) can be of at least 10%, at least 20%, at least 50%, at least at least 80% or at least 90%; it can also be of at least 95%, at least 98% or at least 99%. Further, mixture (II) can consist essentially of or can consist of the aryl aliphatic carbinol of formula (D) and the dialiphatic carbinol of formula (E).

In the **third step** of the process, the mixture containing the dialiphatic carbinol and the aryl aliphatic carbinol undergoes a polyalkoxylation by which polyalkoxy groups are added to the hydroxyl group of both carbinols. The polyalkoxy groups may be polyethoxy groups or polypropoxy groups. The polyalkoxy groups may comprise polyethoxy and polypropoxy groups. In one embodiment, the polyalkoxy groups consist of a block copolymer of ethylene oxide and propylene oxide units. In another embodiment, the polyalkoxy groups consist of a random copolymer of ethylene oxide and propylene oxide units.

Alkoxylation of an alcohol is a process known to the person skilled in the art. Nevertheless, suitable alkoxylation conditions are provided hereafter for indication purposes.

The alkoxylation step is generally preceded by a dehydration step in presence of a base, such as NaOH. The molar percentage of the base amounts generally to less than 10 % of the sum of the number of moles of the two carbinols. The molar percentage of the base can be less than 1% with respect to the sum of the number of moles of the aryl aliphatic carbinol and dialiphatic carbinol. The molar percentage of the base can be less than 5% with respect to the sum of the number of moles of the aryl alkyl carbinol and the dialiphatic carbinol.

Dehydration occurs at a temperature generally ranging from 100 to 150°C, preferably from 120 to 130°C at a pressure which is less than the atmospheric pressure, for example 0.1 bar. Dehydration is carried out for a duration generally ranging from 0.25 to 2 hours.

Then polyalkoxylation is carried out. The required quantity of alkylene oxide is added to the reaction mixture. Alkoxylation is carried out a temperature generally ranging from 100 to 250°C, preferably from 100 to 200°C, preferably from 100 to 150°C and preferably from 120 to 130°C.

In one preferred embodiment, the reaction is started at 125°C during from 2 to 4 hours. This corresponds to

the reaction stage. Then, the temperature is increased at about 130°C during 0.5 to 1.5 hours in order to consume all the alkylene oxide introduced. This corresponds to a post-reaction stage.

A final stage of neutralization is then carried out at a temperature typically ranging from 30°C to 100°C during from 0.5 to 2 hours by addition of a weak acid, such as acetic acid, to the reaction mixture. The molar percentage of the weak acid amounts generally to less than 10 % of the sum of the number of moles of the ethoxylated carbinols.

The third step of the process leads to mixture (I) comprising a mixture of an aryl aliphatic carbinol alkoxylate and a dialiphatic carbinol alkoxylate.

In mixture (I), the weight of the dialiphatic carbinol alkoxylate of formula (B) based on the total weight of the aryl aliphatic carbinol alkoxylate of formula (A) and of the dialiphatic carbinol alkoxylate of formula (B), namely the ratio $r_{BA}$ with $r_{BA}$ being defined as follows:

$$r_{BA} = \text{weight of (B)} / [\text{ weight of (A)} + \text{weight of (B) }]$$

can be of up to 2%, up to 5%, up to 10%, up to 20%, up to 30% or even up to 50%.

Besides, the ratio $r_{BA}$ can be of at least 1%, at least 2%, at least 5%, at least 10% or at least 20%.

Some preferred ranges for $r_{BA}$ are from 1% to 50%, from 1% to 30%, from 2% to 20%, from 2% to 10% and from 5% to 20%.

In mixture (I), the total weight of the aryl aliphatic carbinol alkoxylate of formula (A) and of the dialiphatic carbinol alkoxylate of formula (B) based on the total weight of the mixture (I) can be of at least 10%, at least 20%, at least 50%, at least at least 80% or at least 90%; it can also be of at least 95%, at least 98% or at least 99%. Further, mixture (I) can consist essentially of or can consist of the aryl aliphatic carbinol alkoxylate of formula (A) and the dialiphatic carbinol alkoxylate of formula (B).

Mixture (I) may further contain residual amounts of unreacted aryl aliphatic carbinol and dialiphatic carbinol from mixture (II), both these carbinols having not been alkoxylated. Mixture (I) may also contain residual amounts of unreacted dialiphatic ketone and aryl aliphatic ketone from mixture (III).

Polyalkoxylation is conducted on the mixture containing both the aryl aliphatic carbinol and the dialiphatic carbinol.

It is worth noting that the invention relies on a key catalytic Piria cross-ketonization and starts from widely available carboxylic acids generating only water and $CO_2$ as by-products, in contrast to the process disclosed in CN 106220478 which uses toxic reactants.

Further, the claimed process contains fewer steps, has a better atom economy and lower E-factor. It is therefore significantly more sustainable and economically viable which is a technical advantage.

It is also worth noting that the process produces a composition comprising a mixture of at least two non-ionic surfactants of different natures. It is therefore different from the one described in CN106220478 which produces a composition containing only the NPE substitute, that is to say the alpha-phenyl alkyl alcohol poly(oxyethylene).

Mixture (I) containing the aryl aliphatic carbinol alkoxylate and the dialiphatic carbinol alkoxylate exhibits surfactant properties. The surfactant basic properties, in particular the Hydrophilic-Lipophilic Balance HLB value, may be adjusted according to the user's needs by selecting the nature and the length of the aliphatic R radical and by selecting the nature and the number of moles of the alkoxy group.

Mixture I may be used in a phytosanitary composition or in a pharmaceutical composition. Phytosanitary and pharmaceutical compositions may be prepared under various forms. One may cite:

- an emulsifiable concentrate comprising at least one active ingredient, a solvent and mixture (I). Preferably, mixture (I) amounts to from 5 to 15%, preferably from 7 to 10 % of the weight of the emulsifiable concentrate.
- a microemulsion comprising at least one active ingredient, water, a co-solvent and mixture (I). Preferably, mixture (I) amounts to from 10 to 40%, preferably from 15 to 30 % of the weight of the microemulsion.
- an emulsion in water comprising at least one active ingredient, water, a co-solvent and mixture (I). Preferably, mixture (I) amounts to from 5 to 15%, preferably from 7 to 10 % of the weight of the emulsion in water.
- a suspension concentrate comprising at least one active ingredient, water, optionally a wetting agent, mixture (I) and at least one additive selected form an antifreeze agent, an anti-foam agent, an antibacterial agent, a thickener. Preferably, the wetting agent amounts to less than 1 % of the weight of the suspension concentrate. Mixture (I) acts as a dispersing agent. In addition to its dispersing properties, mixture (I) can act also as a wetting agent. Preferably, mixture (I) amounts to from 2 to 10%, preferably from 3 to 6% of the weight of the suspension concentrate.

In all of the above forms, the solvent or co-solvent may be an organic solvent, preferably originating from the refinery of crude oil. One may cite xylene and naphtha.

The emulsifiable concentrate, the microemulsion, the emulsion and the suspension comprise may optionally

further comprise a co-surfactant. The co-surfactant is advantageously an anionic surfactant; it is preferably chosen from optionally alkoxylated alkyl sulfates, optionally alkoxylated alkyl sulfonates, optionally alkoxylated alkylaryl sulfates, optionally alkoxylated alkylaryl sulfonates and mixtures thereof, and is more preferably an alkylaryl sulfonate (understand: an alkylaryl sulfonate which is not alkoxylated); the counter-ion of the anionic surfactant, in particular of the alkylaryl sulfonate, can be an ammonium cation, an alkali metal cation or an alkaline-earth metal cation. As example of alkylaryl sulfonate, it can be cited linear calcium dodecylbenzene sulfonate, commercially available as Rhodacal® 60/B from RHODIA OPERATIONS. The amount of co-surfactant, based on the total weight of the emulsifiable concentrate, the microemulsion, the emulsion and the suspension, as the case may be, ranges generally from 0.1% to 10%, in particular from 1% to 5%.

The present invention will now be illustrated by the following examples, which are not intended to be limiting.

**EXAMPLES**

Examples of the synthesis of alkoxylate compositions are described hereafter:
All the reactions were conducted under a strictly oxygen-free atmosphere (under argon).

**A) Cross-ketonization:**

**1) From benzoic acid and $C_{16}$ fatty acid:**

**1.1) Using 0.37 equivalent of benzoic acid and 1 equivalent of palmitic acid (55:45 ratio):**

In a round bottom flask equipped with a Dean-Stark apparatus, an insulated addition funnel filled with 50g of melted palmitic acid (195.1 mmol), a second solid addition funnel filled with 8.6g of benzoic acid (70.5 mmol), a temperature probe and a mechanical stirrer are added 2g of iron (35.8 mmol).

- Initial complex generation and decomposition:

Then, 12.5g (48.8 mmol) of palmitic acid followed by 2.9g (23.8 mmol) of benzoic acid are added into the reaction medium and the mixture is allowed to stir at 250°C during 2h. During this 1st stage, $Fe^{(0)}$ is converted to a mixture of $Fe^{(II)}$ carboxylate complexes with concomitant release of hydrogen gas. After complete conversion of acids toward complexes (that can be followed thanks to FTIR analysis), the reaction media is heated to 315°C and allowed to stir at this temperature during 1h30 in order to complete the transformation of the intermediates complexes containing at least one aliphatic carboxylate ligand toward the corresponding ketones. Release of $CO_2$ gas is observed at this stage.

- Cycles of acid addition and ketonization:

Thereafter, 12.5g of palmitic acid (48.8 mmol) and 1.9g of benzoic acid (15.6 mmol) are added into the reactor and the mixture allowed stirring at 315°C. The reaction progress can be followed by FTIR analysis which showed that conversion of the complexes toward ketones was achieved after 1h15 of reaction time after the end of the addition. Two additional cycles each consisting on acids addition (12.5g of palmitic acid and 1.9g of benzoic acid) followed by heating at 315°C during 1h15 were performed.

Finally, the reaction progress was monitored thanks to FTIR in order to ensure that complete conversion of complexes was reached. This requires usually an additional 0h30 of stirring after the last cycle.

- Work-up:

After reaction completion, the reaction mixture is cooled down at 40°C and chloroform ($CHCl_3$) is added into the mixture to dissolve products. In order to separate the desired product from iron oxide and remaining iron carboxylate complex, the chloroform mixture is filtered over a silica plug and the product eluted with around 1.5 L of $CHCl_3$. After evaporation of the solvent, 47.71 g of product (91% yield relative to fatty acids) is obtained as a light brown powder constituted of 55 mol % of cross-adduct phenylhexadecan-1-one and 45 mol % of hentriacontan-16-one (palmitone).

**1.2) Using 0.83 equivalent of benzoic acid and 1 equivalent of palmitic acid (85:15 ratio):**

The reaction is carried out following the protocol described in paragraph 1.1 with a total amount of 50g of palmitic acid (195.1 mmol), 19.8g of benzoic acid (162.2 mmol) and 3.4g of $Fe^{(0)}$ (60.8 mmol).

During initial complex generation and decomposition, 3.4g (60.8 mmol) of Fe is reacted with 12.5g (48.8 mmol)

of palmitic acid and 9.0g (73.7 mmol) of benzoic acid.

During the 3 cycles of acids addition and decomposition, 12.5g (48.8 mmol) of palmitic acid and 3.6g (29.5 mmol) of benzoic acid are added at each cycle.

The product is obtained as a brown powder (51.84 g, 91% isolated yield relative to the fatty acid) and consists of 85 mol % of cross adduct and 15 mol % of hentriacontan-16-one.

**1.3) Using 1.75 equivalent of benzoic acid and 1 equivalent of palmitic acid (99:1 ratio):**

The reaction is carried out following the protocol described in paragraph 1.1 with a total amount of 32.8g of palmitic acid (128.0 mmol), 27.2g of benzoic acid (222.9 mmol) and 4.9g of Fe (87.6 mmol).

During initial complex generation and decomposition, 4.9g (87.6 mmol) of Fe is reacted with 8.2g (32.0 mmol) of palmitic acid and 17.6g (144.2 mmol) of benzoic acid.

During the 3 cycles of acids addition and decomposition, 8.2g (32.0 mmol) of palmitic acid and 3.2 g (26.2 mmol) of benzoic acid are added at each cycle.

The product is obtained as a brown powder (33.75 g, 83% isolated yield relative to the fatty acid) and consists of 99 mol % of cross adduct and only 1 mol % of hentriacontan-16-one.

**2) From benzoic acid and $C_{12}$ fatty acid:**

**2.1 Using 0.83 equivalent of benzoic acid and 1 equivalent of lauric acid (87:13 ratio):**

The reaction is carried out following the protocol described in paragraph 1.1 with a total amount of 39.2g of lauric acid (195.8 mmol), 19.8g of benzoic acid (162.2 mmol) and 3.4g of $Fe^{(0)}$ (60.8 mmol).

During initial complex generation and decomposition, 3.4g (60.8 mmol) of Fe is reacted with 9.8g (48.9 mmol) of lauric acid and 9.0g (73.7 mmol) of benzoic acid.

During the 3 cycles of acids addition and decomposition, 9.8g (48.9 mmol) of lauric acid and 3.6g (29.5 mmol) of benzoic acid are added at each cycle.

The product is obtained as a brown powder (40.3g, 87% isolated yield relative to the fatty acid) and consists of 87 mol % of cross adduct and 13 mol % of tricosan-12-one.

**2.2 Using 1.75 equivalent of benzoic acid and 1 equivalent of lauric acid (99:1 ratio):**

The reaction is carried out following the protocol described in paragraph 1.1 with a total amount of 25.6g of lauric acid (127.9 mmol), 27.2g of benzoic acid (222.9 mmol) and 4.9g of $Fe^{(0)}$ (87.6 mmol).

During initial complex generation and decomposition, 4.9g (87.6 mmol) of Fe is reacted with 6.4g (31.9 mmol) of lauric acid and 17.6g (144.2 mmol) of benzoic acid.

During the 3 cycles of acids addition and decomposition, 6.4g (31.9 mmol) of lauric acid and 3.2g (26.2 mmol) of benzoic acid are added at each cycle.

The product is obtained as a brown powder (23.84g, 71% isolated yield relative to the fatty acid) and consists of 99 mol % of cross adduct and 1 mol % of tricosan-12-one.

**B) Hydrogenation:**

The hydrogenation reactions are conducted in 35 mL or 100 mL autoclaves (Hastelloy C22) equipped with a self-aspirating Rushton turbine to ensure good transfer.

*General protocol for the hydrogenation of 1-phenyldodecan-1-one + 12-tricosanone mixture:*

The starting mixture of ketones (solid in the form of a powder) is mixed with a NaOH powder and Pd/C (3% of Pd on a carbon support) and is then loaded into the reactor. The reactor is then purged 3 times with nitrogen and 3 times with hydrogen. The reactor is pressured under 20 bar of hydrogen and the temperature is set at 80°C in order to melt the ketones mixture.

When the temperature of the reaction mixture reaches 80°C, stirring can be started at a speed of 1000 rpm and the temperature is then increased to the desired reaction temperature (cf table 1 below). The reaction mixture is then allowed to stir at this temperature under 20 bar of hydrogen during several hours. Then the reactor is cooled down at room temperature, the pressure is decreased down to 1 bar and 70 mL of $CHCl_3$ are added. The suspension is filtered in order to remove the catalyst and the solvent is evaporated under vacuum to yield a white solid which is then analyzed thanks to NMR in order to quantify conversion & selectivity.

The following table summarizes the impact of some reaction parameters (Temperature, NaOH concentration, reaction time) on the performances of the reaction.

All the reactions were conducted using 3 wt.% of a Pd/C catalyst (the mass percentage of carbon being 3% with respect to the mass of the catalyst).

TT is the conversion rate of the ketone and is defined as TT=($n_i$(ketone) - $n_f$ketone))/$n_i$(ketone) $\times$ 100 where $n_i$(ketone) is the initial number of moles of ketone introduced into the reactor and $n_f$(ketone) is the remaining molar amount of ketone after the reaction.

RT indicates the selectivity toward the carbinol and is defined as

$$RT = n_f(\text{carbinol})/(n_i(\text{ketone}) - n_f(\text{ketone})) \times 100$$

where $n_f$(carbinol) is the number of moles of carbinol formed through reduction of the corresponding ketone, $n_i$(ketone) and $n_f$(ketone) are defined as above.

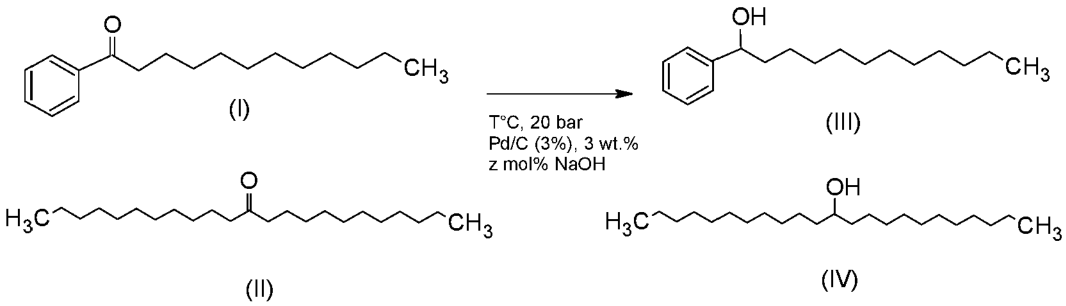


(I) (II) (III) (IV)

Table 1

| (I):(II) wt.ratio | T (°C) | P $H_2$ (bar) | Reaction time (h) | NaOH (mol%) | TT (I) | RT (III) | TT (II) | RT (IV) |
|---|---|---|---|---|---|---|---|---|
| 100:0 | 90 | 20 | 3h00 | 5 | 98 | 99 | - | - |
| 95:5 | 90 | 20 | 6h20 | 5 | 88 | 100 | 0 | - |
| 85:15 | 90 | 20 | 5h00 | 5 | 96 | 97 | 0 | - |
| 50:50 | 90 | 20 | 4h30 | 5 | 99 | 94 | 0 | - |
| 95:5 | 120 | 20 | 4h00 | 5 | 98 | 97 | 9 | 100 |
| 85:15 | 120 | 20 | 4h00 | 5 | 99 | 96 | 10 | 100 |
| 50:50 | 120 | 20 | 4h00 | 5 | 99 | 79 | 7 | 100 |
| 95:5 | 150 | 20 | 4h00 | **5** | **98** | **95** | **24** | 100 |
| 95:5 | 150 | 20 | 4h00 | **10** | **99** | **94** | **53** | 100 |
| 95:5 | 150 | 20 | 4h00 | **15** | **97** | **95** | **60** | 100 |
| 85:15 | 150 | 20 | 4h00 | **15** | **94** | **98** | **73** | 100 |
| 85:15 | 150 | 20 | 4h00 | **15** | **96** | **98** | **71** | 100 |
| 85:15 | 150 | 20 | 4h00 | **15** | **97** | **97** | **69** | 100 |

As shown on table 1 above, the conditions that afford both good conversion and selectivity are: 150°C, 20 bar, 15 mol % of NaOH (with respect to the sum of the number of moles of ketones) and Pd/C (3%): 3 wt.%.

Starting from a mixture of 1-phenyldodecan-1-one and 12-tricosanone in the weight ratio 85:15 and applying these conditions, one obtains after 4 hours a conversion rate of 1-phenyldodecan-1-one of 97% (selectivity toward the carbinol is 97%) and a conversion rate of 12-tricosanone of 70%, with very good selectivity toward 12-tricosanol.

**C) Alkoxylation:**

**1) Ethoxylation of 1-phenyldodecan-1-ol (average molar ratio EO : alcohol = 10)**

The NaOH addition and dehydration steps are conducted in a standard glassware equipment. 3.3 g ofNaOH 50 wt.% aqueous solution (0.04 mole) is reacted with 985 g (3.75 moles) of 1-phenyldodecan-1-one at 125°C under reduced pressure (0.1 bar) during 30 minutes. The residual amount of water after this step is 176 ppm.
The ethoxylation reaction is then conducted in a 15 L spray loop reactor (stainless steel) equipped with a heat exchanger.
The reaction is started at 125°C during 3h and then the temperature is increased at 130°C during 1h. During this reaction phase, the pressure goes from 0.5 barg to 4.5 barg, the average EO flow rate is around 400 g/h with a maximum of 750 g/h.
A post-reaction step is then conducted at 130°C during 1h00 in order to consume totally the ethylene oxide. During this post-reaction stage the pressure drops from 4.5 barg to 1.0 barg.
In total, 1654 g of ethylene oxide has been reacted (37.59 moles).
The final neutralization stage is again conducted in a standard glassware equipment at 50°C using 3.0 g of acetic acid 80 wt.% (0.04 mole).
The obtained product is liquid at room temperature and exhibits the following properties:

- pH of a 5% solution in water = 5.6
- cloud point at 10% in butyl diglycol (BDG) 25% = 73.5°C
- hydroxyl value = 82 mg KOH/g

According to the NMR spectroscopy technique, the products consists of a mixture composed of 97 wt. % of ethoxylated product (average a number of EO around 10) and 3 wt.% of residual alcohol precursor.
The HPLC analysis allowed determining the amount of free polyethylene glycol (PEG): 5.7 % (equivalent PEG400).

**2) Ethoxylation 12-tricosanol (average molar ratio EO: alcohol = 10)**

The NaOH addition and dehydration steps are conducted in a standard glassware equipment.
5.1 g ofNaOH 50 wt.% aqueous solution (0.06 mole) is reacted with 1000 g (2.94 moles) of 12-tricosanol at 125°C under reduced pressure (0.1 bar) during around 30 minutes. The residual amount of water after this step is 180 ppm.
The ethoxylation reaction is then conducted in a 15L spray loop reactor (stainless steel) equipped with a heat exchanger.
The reaction is started at 125°C and 2 equivalents of ethylene oxide are added. The temperature is then increased to 150°C and the 8 remaining equivalents of ethylene oxide are added.
The post-reaction step is conducted at 150°C in order to consume totally the ethylene oxide.
In total, 1293 g of ethylene oxide has been reacted (29.41 moles)
The final neutralization stage is conducted in a standard glassware equipment at 80°C using 5.0 g of isononanoic acid (0.03 mole).
The obtained product is solid at room temperature and exhibits the following properties:

- pH 5% hydro-alcoholic = 6.8
- cloud point at 10% in BDG 25% = 79.5°C
- hydroxyl value = 70.8 mg KOH/g.

According to NMR spectroscopy technique the product consists on a mixture composed of 71 wt. % of ethoxylated secondary alcohol (average number of EO around 21) and 29 wt.% of the alcohol precursor.
HPLC analysis allowed determining the amount of free PEG: 490 ppm (equivalent PEG400).

**D) Emulsification of agriculture formulations for different blends comprising the ethoxylation products of 1-phenyldodecan-1-ol and 12-tricosanol:**

The ethoxylated 1-phenyldodecan-1-ol (10 EO) was blended with the product resulting from the ethoxylation of 12-tricosanol (10 EO) at different weight ratios. The various weight ratios are indicated in Table 2.

Table 2

| Blend of surfactants n° | Weight percentage of 1-phenyldodecan-1-ol (+10 EO) in the blend | Weight percentage of 12-tricosanol (+10 EO) in the blend |
|---|---|---|
| 1 | 100 | 0 |
| 2 | 98 | 2 |
| 3 | 90 | 10 |
| 4 | 80 | 20 |

The ability of the various blends to emulsify some agro pesticides in the presence of an anionic co-surfactant, such as dodecylbenzene sulfonate derivatives, was assessed. In this view, various emulsifiable concentrates (EC) were prepared. The composition of the various emulsifiable concentrates are indicated in Table 3.

Table 3

| EC | Agro pesticide | | Blend of surfactants | | Co-surfactant | | Solvent | |
|---|---|---|---|---|---|---|---|---|
| 1 | Cypermethrin tech.96 % | 26.1% | n°1 | 10.5% | Rhodacal® 60/B | 4.5% | xylene | 58.9% |
| 2 | Chlorpyrifos tech. 98% | 45.9% | n°1 | 3.9% | Rhodacal® 60/B | 3.1% | Solvesso™ 150-ND | 47.1% |
| 3 | Chlorpyrifos tech. 98% | 46% | n°2 | 3.9% | Rhodacal® 60/B | 3.1% | Solvesso™ 150-ND | 47% |
| 4 | Chlorpyrifos tech. 98% | 46% | n°3 | 3.9% | Rhodacal® 60/B | 3.1% | Solvesso™ 150-ND | 47% |
| 5 | Chlorpyrifos tech. 98% | 46% | n°4 | 3.9% | Rhodacal® 60/B | 3.1% | Solvesso™ 150-ND | 47% |
| Rhodacal® 60/B is a linear calcium dodecylbenzene sulfonate, thus an anionic surfactant Solvesso™ 150-ND is a solvent naphtha | | | | | | | | |

**1) Assessment of the ability of the non-ionic surfactants of the present invention to emulsify the agro pesticides:**

The tests for assessing the stability of the oil-in-water emulsions were carried out according to the procedure as defined in the first version of the Cipac MT 36 international standard entitled *"Emulsion Characteristics of emulsifiable concentrates"*. The composition of the standard waters A, D and C used in the tests is defined in the Cipac MT 18 international standard.

The time after which the stability was assessed was set to 2 hours. The emulsification tests were run at 30°C. The concentration of the emulsion in water was 5% v/v. The results are summarized in Table 4.

Table 4

| | A=20 ppm | D=342 ppm | C=500 ppm |
|---|---|---|---|
| EC1 | 0.5 | 0 | t |
| EC2 | 0.6 | 0 | t |
| EC3 | 1 | t | 0 |
| EC4 | 0.8 | 0 | t |
| EC5 | 1 | t | 0 |

The stability of the emulsion was assessed using the following rating:

"1" means phase separation,
"0" means no phase separation (white solution) and
"t" means traces of cream.

The results above show that the ethoxylate compositions that can be obtained in 3 steps from carboxylic acids

through:

1) cross-ketonization between benzoic acid and a fatty acid
2) hydrogenation of the ketones mixture and
3) carbinols mixture ethoxylation

are promising candidates for the emulsification of agrochemicals.

**2) Measurement of surface tension in water:**

The blends of surfactants 1-4 were added to water at a concentration of 0.1 wt.%. The surface tension was then measured. The results are summarized in Table 5.

Table 5

| Blend of surfactants n° | Surface tension (dyne/cm) |
|---|---|
| 1 | 30.1 |
| 2 | 31.5 |
| 3 | 32 |
| 4 | 32 |
| reference (no surfactants) | ~ 70 |

The surface tension in the absence of any surfactants is about 70 dyne/cm. Surface tension measurement show that the compositions containing the blend of surfactants give surface tension values in the range of 30-32 dyne/cm for all the studied ratios, thus lower than 70 dyne/cm. Those low surface tensions are indicative of good wetting properties of the composition.

**Claims**

**1.** A process for the preparation of a mixture (I) comprising:

- an aryl aliphatic carbinol alkoxylate of formula (A):

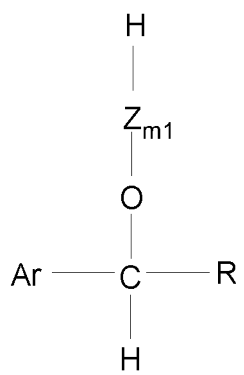

(A)

where:

R is an aliphatic radical having from 4 to 24 carbon atoms;
Z is an alkoxylate unit of general formula -(Y-O)- where Y is a divalent hydrocarbon radical containing from 2 to 6 carbon atoms and 50≥m1≥2;
Ar is an aryl radical;

- a dialiphatic carbinol alkoxylate of formula (B):

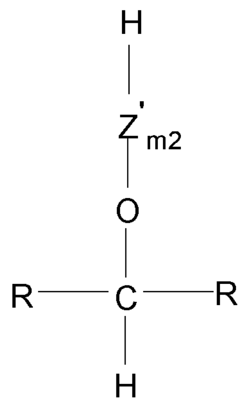
(B)

where

R is the same as above;
Z' is an alkoxylate unit of general formula -(Y'-O)- where Y' is a divalent hydrocarbon radical containing from 2 to 6 carbon atoms where Z' can be the same as Z or can be different from Z and 50≥m2≥2
said process comprising the steps of:

a) performing a catalytic decarboxylative cross-ketonisation of aryl- and aliphatic carboxylic acids comprising the steps of:

i) providing a mixture containing:

- an aryl carboxylic acid of formula

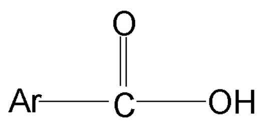

where Ar is the same as defined above;
- an aliphatic carboxylic acid of formula

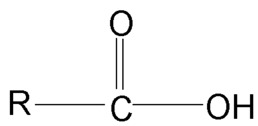

where R is the same as defined above;
- a catalyst;

ii) heating the mixture at a temperature sufficient to form mixture (III) comprising a dialiphatic ketone of formula (F)

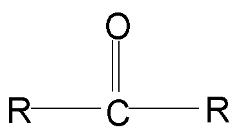
(F)

where R is the same as defined above
and an aryl aliphatic ketone of formula (G)

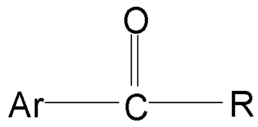 (G)

where R and Ar are the same as defined above;

b) subjecting mixture (III) to a hydrogenation step in the presence of a base to form mixture (II) comprising:

- an aryl aliphatic carbinol of formula (D):

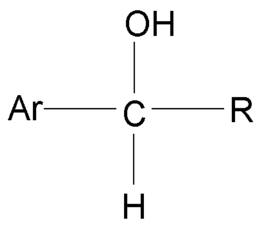 (D)

where

R is an aliphatic radical having from 4 to 24 carbon atoms;
Ar is an aryl radical;

- a dialiphatic carbinol of formula (E):

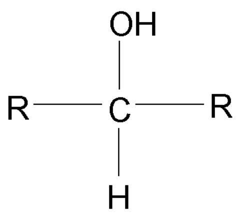 (E)

where R is the same as defined above;

c) subjecting mixture (II) to an alkoxylation step to form mixture (I);

wherein step a) of performing the catalytic decarboxylative cross-ketonisation of aryl- and aliphatic carboxylic acids comprises the steps of:

i) providing a mixture containing:

- the aryl carboxylic acid;
- the aliphatic carboxylic acid;
- a metal-containing compound;

in which the number of moles of the metal in the mixture is at least equal to 90% of the sum of the number of moles of aryl carboxylic acid and the number of moles of aliphatic carboxylic acid divided by the valency of the metal
said mixture being free of any added solvent;

ii) heating the mixture at a temperature sufficient to form metal carboxylates;
iii) further heating the mixture at a temperature sufficient to form the dialiphatic ketone of formula (F) and the aryl aliphatic ketone of formula (G);

iv) adding to the reaction mixture of step iii):

- aryl carboxylic acid in an amount which corresponds substantially to the amount of aryl carboxylic acid consumed during the formation of the aryl aliphatic ketone in step iii);
- aliphatic carboxylic acid in an amount which corresponds substantially to the amount of aliphatic carboxylic acid consumed during the formation of the aryl aliphatic ketone and the dialiphatic ketone in step iii);

maintaining the mixture at a temperature sufficient to continue forming the dialiphatic ketone and the aryl aliphatic ketone;
v) optionally repeating step iv).

**2.** The process according to claim 1, wherein in mixture (I) the weight of the dialiphatic carbinol alkoxylate of formula (B) based on the total weight of the aryl aliphatic carbinol alkoxylate of formula (A) and of the dialiphatic carbinol alkoxylate of formula (B), ranges from 2% to 20%.

**3.** The process according to claim 1 or 2, wherein in mixture (I), the aliphatic radical is an alkyl radical.

**4.** The process according to anyone of claims 1 to 3, wherein:

- the polyalkoxylated chain $-Z_{m1}-$ has formula (C) where $0 \leq x \leq 20$ ; $0 \leq y \leq 20$ and $2 \leq x+y \leq 40$;

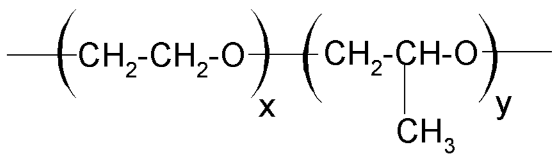

(C)

- the polyalkoxylated chain $-Z'_{m2}-$ has formula (C') where $0 \leq x' \leq 20$ ; $0 \leq y' \leq 20$ and $2 \leq x'+y' \leq 40$ ;

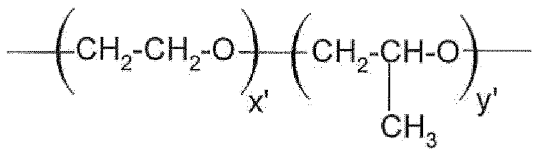

(C')

.

**5.** The process according to any one of claims 1 to 4, wherein the aliphatic carboxylic acid is selected from the group consisting of caprylic acid ($C_8$), capric acid ($C_{10}$), lauric acid ($C_{12}$), myristic acid ($C_{14}$), palmitic acid ($C_{16}$), oleic and stearic acids ($C_{18}$).

**6.** The process according to any one of claims 1 to 4, wherein the aryl carboxylic acid is selected from the group consisting of benzoic acid, toluic acid, furoic acid, nicotinic acid, thiophenecarboxylic acid, 2-mesitylenecarboxylic acid, isopropylbenzoic acid, chlorobenzoic acid, fluorobenzoic acid, trifluoromethylbenzoic acid, methoxybenzoic acid, naphthoic acid, anthracenecarboxylic acid, biphenylcarboxylic acid, benzoylbenzoic acid, preferably benzoic acid and toluic acid.

## Patentansprüche

**1.** Verfahren zur Herstellung eines Gemischs (I), umfassend:

- ein arylaliphatisches Carbinolalkoxylat der Formel (A):

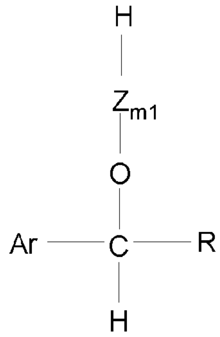

(A)

wobei:

R für einen aliphatischen Rest mit 4 bis 24 Kohlenstoffatomen steht;
Z für eine Alkoxylat-Einheit der allgemeinen Formel -(Y-O)- steht, wobei Y für einen zweiwertigen Kohlenwasserstoffrest mit 2 bis 6 Kohlenstoffatomen steht und 50 ≥ m1 ≥ 2;
Ar für einen Arylrest steht;

- ein dialiphatisches Carbinolalkoxylat der Formel (B) :

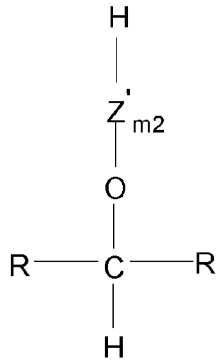

(B)

wobei

R wie oben definiert ist;
Z' für eine Alkoxylat-Einheit der allgemeinen Formel -(Y'-O)- steht, wobei Y' für einen zweiwertigen Kohlenwasserstoffrest mit 2 bis 6 Kohlenstoffatomen steht, wobei Z' mit Z identisch oder davon verschieden sein kann, und 50 ≥ m2 ≥ 2;
wobei das Verfahren die folgenden Schritte umfasst:

a) Durchführen einer katalytischen decarboxylierenden Kreuzketonisierung von Aryl- und aliphatischen Carbonsäuren, umfassend die Schritte:

i) Bereitstellen eines Gemischs, enthaltend

- eine Arylcarbonsäure der Formel

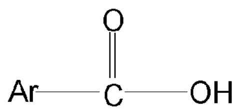

wobei Ar wie oben definiert ist;
- eine aliphatische Carbonsäure der Formel

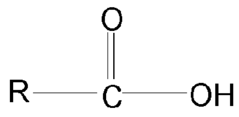

wobei R wie oben definiert ist;
- einen Katalysator;

ii) Erhitzen des Gemischs auf eine Temperatur, die ausreicht, um das Gemisch (III) zu bilden, das ein dialiphatisches Keton der Formel (F)

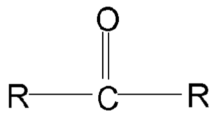 (F)

wobei R wie oben definiert ist,
und ein arylaliphatisches Keton der Formel (G)

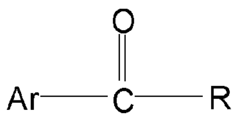 (G)

wobei R und Ar wie oben definiert sind, umfasst;

b) Hydrieren des Gemischs (III) in Gegenwart einer Base zur Bildung des Gemischs (II), umfassend

- ein arylaliphatisches Carbinol der Formel (D):

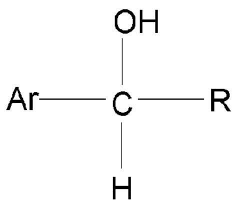 (D)

wobei

R für einen aliphatischen Rest mit 4 bis 24 Kohlenstoffatomen steht;
Ar für einen Arylrest steht;

- ein dialiphatisches Carbinol der Formel (E):

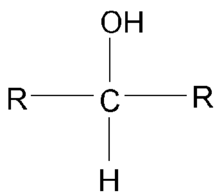 (E)

wobei R wie oben definiert ist;

c) Alkoxylieren des Gemischs (II) zur Bildung des Gemischs (I);

wobei der Schritt a) des Durchführens der katalytischen decarboxylierenden Kreuzketonisierung von Aryl-

und aliphatischen Carbonsäuren die folgenden Schritte umfasst:

i) Bereitstellen eines Gemischs, enthaltend:

- die Arylcarbonsäure;
- die aliphatische Carbonsäure;
- eine metallhaltige Verbindung;

wobei die Molzahl des Metalls in dem Gemisch mindestens gleich 90 % der Summe der Molzahl an Arylcarbonsäure und der Molzahl an aliphatischer Carbonsäure, geteilt durch die Wertigkeit des Metalls, ist,
wobei die Mischung frei von zugegebenem Lösungsmittel ist;

ii) Erhitzen des Gemischs auf eine Temperatur, die ausreicht, um Metallcarboxylate zu bilden;
iii) weiteres Erhitzen des Gemischs auf eine Temperatur, die ausreicht, um das dialiphatische Keton der Formel (F) und das arylaliphatische Keton der Formel (G) zu bilden;
iv) Zugeben von

- Arylcarbonsäure in einer Menge, die weitgehend der Menge an Arylcarbonsäure entspricht, die bei der Bildung des arylaliphatischen Ketons in Schritt iii) verbraucht wird;
- aliphatischer Carbonsäure in einer Menge, die weitgehend der Menge an aliphatischer Carbonsäure entspricht, die bei der Bildung des arylaliphatischen Ketons und des dialiphatischen Ketons in Schritt iii) verbraucht wird;

zu dem Reaktionsgemisch aus Schritt iii);
wobei das Gemisch bei einer Temperatur gehalten wird, die ausreicht, um die Bildung des dialiphatischen Ketons und des arylaliphatischen Ketons fortzusetzen;

v) gegebenenfalls Wiederholen von Schritt iv).

**2.** Verfahren nach Anspruch 1, wobei in dem Gemisch (I) das Gewicht des dialiphatischen Carbinolalkoxylats der Formel (B), bezogen auf das Gesamtgewicht des arylaliphatischen Carbinolalkoxylats der Formel (A) und des dialiphatischen Carbinolalkoxylats der Formel (B), im Bereich von 2 bis 20 % liegt.

**3.** Verfahren nach Anspruch 1 oder 2, wobei in dem Gemisch (I) der aliphatische Rest ein Alkylrest ist.

**4.** Verfahren nach einem der Ansprüche 1 bis 3, wobei:

- die polyalkoxylierte Kette $-Z_{m1}-$ die Formel (C) aufweist, wobei $0 \leq x \leq 20$; $0 \leq y \leq 20$ und $2 \leq x + y \leq 40$;

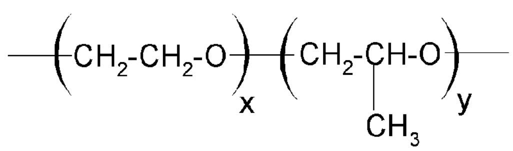 (C)

- die polyalkoxylierte Kette $-Z'_{m2}-$ die Formel (C') aufweist, wobei $0 \leq x' \leq 20$; $0 \leq y' \leq 20$ und $2 \leq x' + y' \leq 40$;

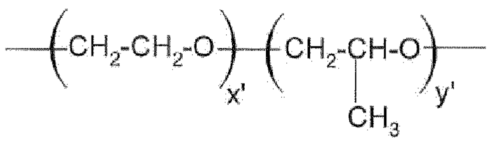 (C')

**5.** Verfahren nach einem der Ansprüche 1 bis 4, wobei die aliphatische Carbonsäure aus der Gruppe bestehend aus Caprylsäure ($C_8$), Caprinsäure ($C_{10}$), Laurinsäure ($C_{12}$), Myristinsäure ($C_{14}$), Palmitinsäure ($C_{16}$), Ölsäure und Stearinsäure ($C_{18}$) ausgewählt wird.

**6.** Verfahren nach einem der Ansprüche 1 bis 4, wobei die Arylcarbonsäure aus der Gruppe bestehend aus Benzoesäure, Toluylsäure, Furoesäure, Nicotinsäure, Thiophencarbonsäure, 2-Mesitylencarbonsäure, Isopropylbenzoesäure, Chlorbenzoesäure, Fluorbenzoesäure, Trifluormethylbenzoesäure, Methoxybenzoesäure, Naphthoesäure, Anthracencarbonsäure, Biphenylcarbonsäure, Benzoylbenzoesäure, vorzugsweise Benzoesäure und Toluylsäure, ausgewählt wird.

## Revendications

**1.** Procédé pour la préparation d'un mélange (I) comprenant :

- un alcoxylate de carbinol arylaliphatique de formule (A) :

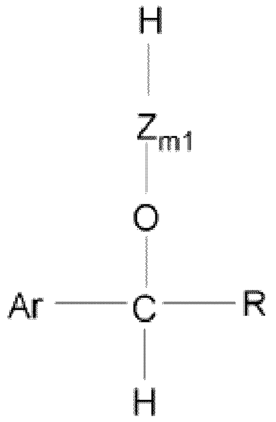

(A)

où :

R est un radical aliphatique ayant de 4 à 24 atomes de carbone ;
Z est un motif alcoxylate de formule générale - (Y-O)- où Y est un radical hydrocarboné divalent contenant de 2 à 6 atomes de carbone et $50 \geq m1 \geq 2$ ;
Ar est un radical aryle ;

- un alcoxylate de carbinol dialiphatique de formule (B) :

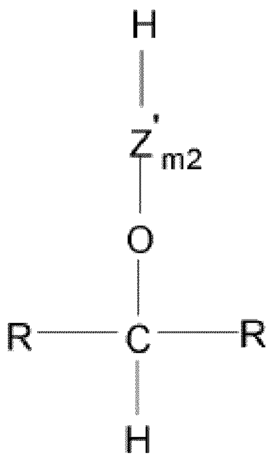

(B)

où

R est le même que ci-dessus ;
Z' est un motif alcoxylate de formule générale - (Y'-O)- où Y' est un radical hydrocarboné divalent contenant de 2 à 6 atomes de carbone, où Z' peut être le même que Z ou peut être différent de Z et $50 \geq m2 \geq 2$
ledit procédé comprenant les étapes de :

a) réalisation d'une cétonisation croisée décarboxylante catalytique d'acides carboxyliques aryliques et aliphatiques comprenant les étapes de

i) fourniture d'un mélange contenant :

- un acide arylcarboxylique de formule

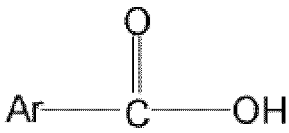

où Ar est le même que défini ci-dessus ;
- un acide carboxylique aliphatique de formule

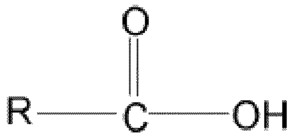

où R est le même que défini ci-dessus ;
- un catalyseur ;

ii) chauffage du mélange à une température suffisante pour former un mélange (III) comprenant une cétone dialiphatique de formule (F)

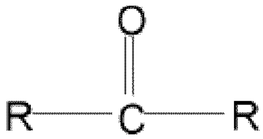 (F)

où R est le même que défini ci-dessus ;
et une cétone arylaliphatique de formule (G)

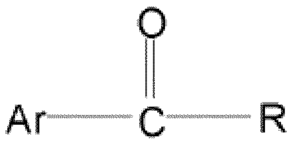 (G)

où R et Ar sont les mêmes que défini ci-dessus ;

b) soumission du mélange (III) à une étape d'hydrogénation en la présence d'une base pour former un mélange (II) comprenant :

- un carbinol arylaliphatique de formule (D) :

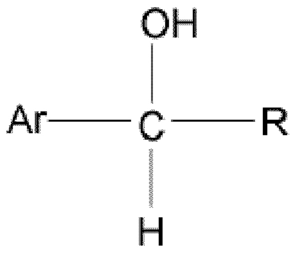

(D)

où

R est un radical aliphatique ayant de 4 à 24 atomes de carbone ;
Ar est un radical aryle ;

- un carbinol dialiphatique de formule (E) :

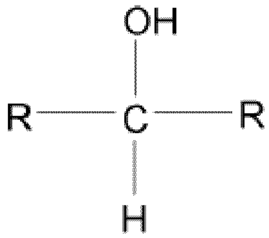

(E)

où R est le même que défini ci-dessus ;

c) soumission du mélange (II) à une étape d'alcoxylation pour former un mélange (I) ;

l'étape a) de réalisation de la cétonisation croisée décarboxylante catalytique d'acides carboxyliques aryliques et aliphatiques comprenant les étapes de :

i) fourniture d'un mélange contenant :

- l'acide arylcarboxylique ;
- l'acide carboxylique aliphatique ;
- un composé contenant un métal ;

dans lequel le nombre de moles du métal dans le mélange est au moins égal à 90 % de la somme du nombre de moles d'acide arylcarboxylique et du nombre de moles d'acide carboxylique aliphatique divisée par la valence du métal
ledit mélange étant exempt d'un quelconque solvant ajouté ;

ii) chauffage du mélange à une température suffisante pour former des carboxylates métalliques ;
iii) chauffage supplémentaire du mélange à une température suffisante pour former la cétone dialiphatique de formule (F) et la cétone arylaliphatique de formule (G) ;
iv) ajout au mélange réactionnel de l'étape iii) :

- d'acide arylcarboxylique en une quantité qui correspond sensiblement à la quantité d'acide arylcarboxylique consommée pendant la formation de la cétone arylaliphatique dans l'étape iii) ;
- d'acide carboxylique aliphatique en une quantité qui correspond sensiblement à la quantité d'acide carboxylique aliphatique consommée pendant la formation de la cétone arylaliphatique et de la cétone dialiphatique dans l'étape iii) ;

maintien du mélange à une température suffisante pour continuer la formation de la cétone dialiphatique et de la cétone arylaliphatique ;
v) éventuellement répétition de l'étape iv).

2. Procédé selon la revendication 1, dans le mélange (I), le poids de l'alcoxylate de carbinol dialiphatique de formule (B) sur la base du poids total de l'alcoxylate de carbinol arylaliphatique de formule (A) et de l'alcoxylate de carbinol dialiphatique de formule (B), se situant dans la plage de 2 % à 20 %.

3. Procédé selon la revendication 1 ou 2, dans le mélange (I), le radical aliphatique étant un radical alkyle.

4. Procédé selon l'une quelconque des revendications 1 à 3,

- la chaîne polyalcoxylée $-Z_{m1}-$ ayant la formule (C) où $0 \leq x \leq 20$ ; $0 \leq y \leq 20$ et $2 \leq x + y \leq 40$ ;

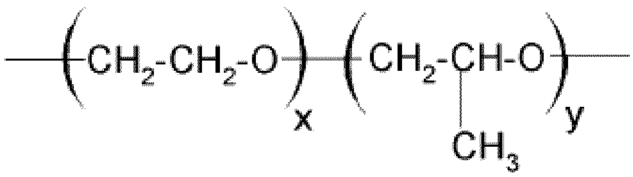

(C)

- la chaîne polyalcoxylée $-Z'_{m2}-$ ayant la formule (C') où $0 \leq x' \leq 20$ ; $0 \leq y' \leq 20$ et $2 \leq x' + y' \leq 40$ ;

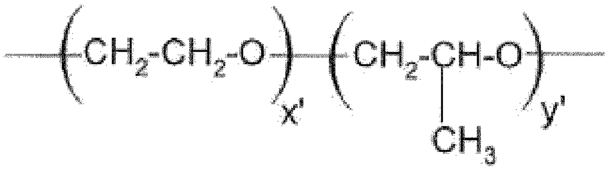

(C')

5. Procédé selon l'une quelconque des revendications 1 à 4, l'acide carboxylique aliphatique étant choisi dans le groupe constitué par l'acide caprylique ($C_8$), l'acide caprique ($C_{10}$), l'acide laurique ($C_{12}$), l'acide myristique ($C_{14}$), l'acide palmitique ($C_{26}$), les acides oléique et stéarique ($C_{28}$).

6. Procédé selon l'une quelconque des revendications 1 à 4, l'acide arylcarboxylique étant choisi dans le groupe constitué par l'acide benzoïque, l'acide toluique, l'acide furoïque, l'acide nicotinique, l'acide thiophènecarboxylique, l'acide 2-mésitylènecarboxylique, l'acide isopropylbenzoïque, l'acide chlorobenzoïque, l'acide fluorobenzoïque, l'acide trifluorométhylbenzoïque, l'acide méthoxybenzoïque, l'acide naphtoïque, l'acide anthracènecarboxylique, l'acide biphénylcarboxylique, l'acide benzoylbenzoïque, préférablement l'acide benzoïque et l'acide toluique.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 17306685
- US 4233174 A
- CN 106220478
- CN 106279670
- CN 106187834
- CN 106220679
- US 6846793 B
- EP 2468708 A